# EUROPÄISCHE PATENTANMELDUNG

(11) **EP 4 628 000 A1**
(43) Veröffentlichungstag der Anmeldung: **08.10.2025**
(21) Anmeldenummer: 24386040.0
(22) Anmeldetag: 03.04.2024
(51) Int. Cl.: A61B 5/00

(54) **MEDIZINISCHE VORRICHTUNG UND VERFAHREN ZUM BETRIEB EINER MEDIZINISCHEN VORRICHTUNG**

(71) Anmelder: Siemens Healthineers AG, 91301 Forchheim (DE)
(72) Erfinder: NUFER, Stephan, 91056 Erlangen, Bayern (DE); HORN, Felix, 90482 Nürnberg (DE); KÜRTEN, Anja, 91056 Erlangen (DE); SPILLER, Barbara, 96050 Bamberg (DE); GIAPITZAKIS, Ioannis-Angelos, 91052 Erlangen (DE); SASANO, Silke, 81827 München (DE)
(74) Vertreter: Siemens Healthineers Patent Attorneys

(57) **Zusammenfassung**

Medizinische Vorrichtung (1), insbesondere medizinische Bildgebungs-, Diagnose- und/oder Behandlungsvorrichtung, umfassend:
- einen Patientenbereich (20), in dem zumindest ein Teil eines Patienten platzierbar ist, und
- eine Überwachungseinrichtung, die eine Sensoreinrichtung (25) umfasst, die dazu ausgelegt ist, eine Untersuchung des Gases im Patientenbereich (20) durchzuführen und ein Ergebnis der Untersuchung als Ausgabeinformation bereitzustellen.

## Beschreibung

Die vorliegende Erfindung betrifft eine medizinische Vorrichtung und ein Verfahren zum Betrieb einer medizinischen Vorrichtung.

Unabhängig vom grammatikalischen Geschlecht eines bestimmten Begriffs sind Personen mit männlicher, weiblicher oder anderer Geschlechteridentität mit umfasst.

Medizinische Vorrichtungen sind hinlänglich bekannt. Zu ihnen gehören beispielsweise medizinische Bildgebungsvorrichtungen, wie MRT- oder CT-Systeme, mit denen beispielsweise ein Körperinneres eines Patienten aufgenommen werden kann. Grundsätzlich gibt es allerdings auch medizinische Vorrichtungen, die zur Diagnose und/oder Behandlung eines Patienten dienen, wie beispielsweise Zahnarztstühle oder vergleichbares. Es hat sich dabei allerdings als nachteilig erwiesen, dass Patienten nach der Behandlung und/oder der Diagnoseuntersuchung oftmals Krankheitserreger an der medizinischen Vorrichtung hinterlassen. Dabei beschränken sich die Kontaminationen in der Regel nicht nur auf Oberflächen, wie beispielsweise Sitz- und/oder Liegeflächen, sondern die Kontamination betrifft ebenfalls eine Raumluft, in der sich der Patient während der Untersuchung und/oder Behandlung aufgehalten hat.

Bei Untersuchungen mit bildgebenden Modalitäten in radiologischen Instituten gibt es somit immer wieder Patienten, die eine bekannte oder unbekannte infektiöse Krankheit haben (z.B. Covid-19, Grippe, etc.), die sich über die Luft, z.B. über Aerosole, überträgt. Typischerweise verbringen diese Patienten längere Zeit im Untersuchungsraum und können die Luft darin kontaminieren, was zu einer Infektion der nachfolgenden Patienten und des Bedienpersonals führen kann.

Zusätzlich können sich Keime und Viren besonders gut in MR-Untersuchungsräumen durch die Luft verbreiten, da im Kontext von MRT-Untersuchungen ein ständiger Luftzug in der Röhre des MRT notwendig ist und die Luft im Gerät und im Raum umgewälzt wird. Dies hängt mit einer Kühlung des Gerätes und des Patientenkörpers zusammen, um unerwünschten Erwärmungen durch applizierte Hochfrequenz entgegenzuwirken. Des Weiteren besteht bei bildgestützten Interventionen in den Untersuchungsräumen von CT/MR/etc., bei z.B. Nadelbiopsien etc. ein Infektionsrisiko durch kontaminierte Luft, weshalb es bspw. in OP-Sälen spezielle Anforderungen an die Luftreinheit gibt.

Bislang versuchen Betreiber von Bildgebungsgeräten, wie z.B. Krankenhäuser, Kliniken und Radiologie-Praxen, durch vorhergehende Tests oder durch das Tragen von Mund-Nase-Schutz-Masken dem Problem entgegenzuwirken. Einige Patienten jedoch tolerieren keine Masken, da sie sich in einem Ausnahmezustand befinden und daher eine gewisse Unklarheit, was die Untersuchung an Ergebnissen bringt, Nervosität, Enge der Röhre mit z.T. Platzangst, Kurzatmigkeit, Atemnot, etc. vorherrschen können. Gerade während eines Aufenthalts im medizinischen Bildgebungsgeräts ist eine Maske der Prozedur oft nicht zuträglich, da bei manchen MRT-Untersuchungen die Luft angehalten werden muss oder z.B. flach geatmet werden muss. Die Maske belastet Patienten hier zusätzlich, die oft durch ihre Krankheitsgeschichte mehr oder weniger beeinträchtigt sind.

Manche Untersuchungsräume werden belüftet, jedoch ist die Leistungs- und Filterfähigkeit von Kunde zu Kunde unterschiedlich und nicht immer ausreichend, zumal sich bildgebende Modalitäten wie MRTs oftmals in den Kellergeschossen befinden. Des Weiteren gibt es auch Luftfilteranlagen. Diese sind jedoch nicht innerhalb eines MRT-Untersuchungsraums aus bekannten Gründen einsetzbar, da sie potenziell magnetisch, und/oder nicht RF- bzw. HF -kompatibel sind.

Während es aus dem Stand der Technik bekannt ist, mit geeigneten Hygienemaßnahmen die Oberflächen von medizinischen Vorrichtungen zu dekontaminieren, erfolgt eine entsprechende Aufwertung der Raumluft in der Regel nicht. Aus der DE 10 2020 216 423 A1 ist es zumindest bekannt, mittels einer UV-Strahlungsquelle innerhalb eines Luftkanals für eine Dekontamination zu sorgen.

Ausgehend vom Stand der Technik macht es sich die vorliegende Erfindung zur Aufgabe, den Komfort des Patienten zu erhöhen und insbesondere dessen Infektionsrisiko zu reduzieren, wenn er eine entsprechende medinische Vorrichtung nutzen bzw. aufsuchen muss.

Die vorliegende Erfindung löst diese Aufgabe mit einem medizinischen Vorrichtung gemäß Anspruch 1 bzw. mit einem Verfahren gemäß Anspruch 11. Weitere Ausführungsbeispiele befinden sich in der Beschreibung, den Unteransprüchen sowie in den Figuren.

Gemäß einem ersten Aspekt ist eine medizinische Vorrichtung, insbesondere medizinische Bildgebungs-, Diagnose- und/oder Behandlungsvorrichtung, vorgesehen, umfassend:
- einen Patientenbereich, in dem zumindest ein Teil eines Patienten platzierbar ist, und
- eine Überwachungseinrichtung, die eine Sensoreinrichtung umfasst, die dazu ausgelegt ist, eine Untersuchung des Gases im Patientenbereich durchzuführen und ein Ergebnis der Untersuchung als Ausgabeinformation bereitzustellen.

Im Gegensatz zum Stand der Technik ist es vorgesehen, dass die Überwachungseinrichtung eine Sensoreinrichtung umfasst, die dazu ausgelegt ist, im Patientenbereich ein Gas zu untersuchen und die Ergebnisse der Untersuchung als Ausgabeinformation bereitzustellen. Dadurch ist es insbesondere möglich, Störstoffe im Gas des Patientenbereichs zu ermitteln. Diese Störstoffe können beispielsweise Krankheitserreger, wie Grippeviren oder COVID-Viren, oder Duftstoffe, die üble Gerüche verursachen, sein. In Reaktion auf eine entsprechende Ausgabeinformation kann dann beispielsweise eine geeignete Gegenmaßnahme veranlasst werden. Z. B. wird die Gasströmung erhöht, um den Patientenbereich von den Störstoffen zu befreien bzw. es wird ein Grad einer Umwälzung des Gases erhöht, wodurch ebenfalls eine Befreiung des Patientenbereichs von Störstoffen bedingt wird. In Reaktion auf die Ausgabeinformation kann mit Vorteil auch ein Warnsignal für ein Bedienpersonal und zur Information über einen Gesundheitszustand, insbesondere einen allgemeinen Gesundheitszustand, des Patienten ausgegeben werden. Alternativ oder ergänzend ist es vorstellbar, dass Maßnahmen zur Desinfektion, insbesondere zum Abtöten von Krankheitserregern und/oder Keimen, im Patientenbereich, insbesondere des Gases im Patientenbereichs, veranlasst oder angepasst werden. Die Sensoreinrichtung kann dabei im Patientenbereich oder außerhalb des Patientenbereichs angeordnet sein. Mit anderen Worten: Die Sensoreinrichtung muss nicht zwingend unmittelbar im Patientenbereich angeordnet sein, sondern das Gas des Patientenbereichs kann auch der Sensoreinrichtung zugeführt werden.

In einer bevorzugt Ausführungsform oder gemäß einem zweiten, die Sensoreinrichtung nicht zwingend umfassenden, Aspekt ist es vorgesehen, dass die medizinische Vorrichtung weiter umfasst:
- eine Strömungseinrichtung, wobei die Strömungseinrichtung dazu ausgelegt ist, eine Gasströmung im Patientenbereich zu ändern und festzulegen und
- eine Steuereinrichtung, die dazu ausgelegt ist, in Abhängigkeit der Ausgabeinformation der Sensoreinrichtung und/oder einer weiteren Ausgabeinformation einer weiteren Überwachungseinrichtung Steuersignale auszugegeben, mit denen die Strömungseinrichtung ansteuerbar ist, um die Gasströmung im Patientenbereich situationsabhängig einzustellen.

Im Gegensatz zu den aus dem Stand der Technik bekannten Vorgehensweisen ist es bevorzugt vorgesehen, dass gezielt mit der Strömungseinrichtung die Gasströmung, d. h. insbesondere eine Größe eines Volumenstroms, im Patientenbereich situationsabhängig eingestellt wird. Dadurch kann gezielt der Komfort des Patienten erhöht bzw. ein Infektionsrisiko des Patienten reduziert werden. Dabei erfolgt die Anpassung der Gasströmung in Abhängigkeit einer Ausgabeinformation, indem in Abhängigkeit von der Ausgabeinformation ein entsprechendes Steuersignal realisiert wird, mit dem die Strömungseinrichtung angesteuert wird bzw. werden kann.

Die Ausgabeinformation wird dabei von einer Überwachungseinrichtung und/oder einer weiteren Überwachungseinrichtung bereitgestellt. Die Überwachungseinrichtung oder die weitere Überwachungseinrichtung kann dabei ein Bestandteil der medizinischen Vorrichtung sein. Es ist auch vorstellbar, dass die Überwachungseinrichtung oder/oder die weitere Überwachungseinrichtung eine externe Vorrichtung ist, die beispielsweise drahtlos ist oder eine Kabelverbindung umfasst, mit der die Steuereinrichtung in kommunikative Verbindung gebracht werden kann bzw. wird. Vorzugsweise weist die Steuereinrichtung eine Schnittstelle zur Kommunikation mit der Überwachungseinrichtung und/oder der weiteren Überwachungseinrichtung auf. Es ist auch vorstellbar, dass die Überwachungseinrichtung und/oder die weitere Überwachungseinrichtung die Kontrollrichtung der medizinischen Vorrichtung ist oder Teil der Kontrollvorrichtung ist, mit der die medizinische Vorrichtung gesteuert und kontrolliert, zum Beispiel zum Einstellen von Funktionsparametern der medizinischen Vorrichtung, wird.

Die Ausgabeinformation und/oder die weitere Ausgabeinformation kann beispielweise eine Messgröße in oder im Umfeld der medizinischen Vorrichtung und/oder ein Parameter der medizinischen Vorrichtung sein, die eingestellt ist oder einstellbar ist, oder eine Patienteninformation, wie beispielsweise dessen SAR-Belastung und/oder Herzrate. Die Ausgabeinformation oder weitere Ausgabeinformation kann somit beispielsweise patientenbezogen sein und/oder raumluftbezogen und/oder das medizinische Gerät bzw. die medizinische Vorrichtung betreffen. Ferner ist es bevorzugt vorgesehen, dass die Steuereinrichtung eine oder mehrere Ausgabeinformationen zur Einstellung der Signalgröße berücksichtigt. Dadurch können mehrere Aspekte bei der Einstellung der Gasströmung berücksichtigt werden. Bevorzugt werden Ausgabeinformationen berücksichtigt, die den Patienten und das Umfeld der medizinischen Vorrichtung, insbesondere die Raumluft, betreffen. Insbesondere versteht der Fachmann unter situationsabhängig, dass das Steuersignal in Abhängigkeit der Ausgabeinformation bestimmt und festgelegt wird, wodurch auch wiederum der Gasstrom eingestellt und insbesondere verändert wird. Dies erfolgt bevorzugt in Echtzeit. Im Falle eines MRT-Geräts ist beispielsweise Folgendes vorstellbar: Verschiedene Bildgebungssequenzen werden mit verschiedenen eingebrachten Energien realisiert, d. h. unter Verwendung unterschiedlicher SAR-Werten, die zur Erwärmung des Körpers führen. Abhängig vom Protokoll (z. B. im Falle SAR-intensiver Sequenzen, die aufeinanderfolgen) könnte der (teils unangenehme) Luftzug gedrosselt oder erhöht werden, um der Erwärmung durch die eingebrachte Energie entgegenzuwirken. Mit anderen Worten: das Steuersignal kennt den Kontext der Untersuchung in Bezug auf die eingebrachte Energie, beispielsweise SAR, und verhält sich dementsprechend.

Hierbei kann eine situationsabhängige Einstellung bzw. Änderung der Gasströmung Folge eines Regelkreises auf Grundlage der Ausgabeinformation sein, wobei der Regelkreis Steuersignale ausgibt, mit denen der Gasstrom erhöht oder verringert wird, um insbesondere eine Geschwindigkeit, das heißt ein Volumenstrom des Gases, im Patientenbereich, derart einzustellen, dass beispielsweise eine beschleunigte Dekontamination des Gases erfolgt, ein beschleunigter Austausch an Störstoffen gegen dekontaminiertes Gas und/oder eine erhöhte Kühlwirkung für den Patienten veranlasst wird. Bevorzugt erfolgt die Einstellung der Gasströmung in Echtzeit. Es ist auch vorstellbar, dass der Regelkreis alternativ oder ergänzend weitere Gegenmaßnahmen veranlasst, wie beispielsweise eine Erhöhung einer Strahlungsdosis beim Abtöten von Keimen.

Beispielsweise umfasst die Strömungseinrichtung einen Ventilator, dessen Rotationsgeschwindigkeit stufenweise oder kontinuierlich abhängig von dem Steuersignal einstellbar ist. Grundsätzlich ist als Strömungseinrichtung jegliche Einrichtung vorstellbar, die dazu geeignet ist, ein Umwälzen des Gases im Patientenbereich zu veranlassen. Insbesondere handelt es sich bei der Gasströmung um eine Luftströmung. Allerdings ist es auch vorstellbar, dass die im Patientenbereich eingelassene Luftströmung mit einem zusätzlichen Duftstoff versehen ist. Daher wird hier von Gasströmung gesprochen, auch wenn es sich bevorzugt im Wesentlichen um die Raumluft im Patientenbereich handelt.

Bevorzugt ist es vorgesehen, dass zur Einstellung der Gasströmung eine Strömungsgeschwindigkeit der Gasströmung veränderbar ist, beispielsweise in dem die Strömungsgeschwindigkeit erhöht oder erniedrigt wird. Es ist auch vorstellbar, dass die Gasströmung in Hinblick auf eine Strömungsrichtung eingestellt wird. Beispielsweise kann gezielt eine Verwirbelung veranlasst werden. Zur Einstellung der Gasströmung kann beispielweise mittels einer Blende ein Teil der Gasströmung blockiert werden oder ein Ventilator hinsichtlich seine Rotationsgeschwindigkeit angepasst werden. Es ist auch vorstellbar, dass ein Querschnitt eines Zuführrohrs angepasst wird. Denkbar ist auch, dass eine zusätzliche Gasströmung abhängig vom Steuersignal hinzugeschaltet wird. Ebenfalls ist es vorstellbar, dass eine Richtung der Gasströmung umgekehrt wird. Beispielsweise wird durch das Steuersignal zumindest zeitweise die Strömungseinrichtung derart eingestellt, dass sie Gas aus dem Patientenbereich ansaugt, statt Gas dem Patientenbereich zuzuführen.

Die Steuereinrichtung reagiert bevorzugt anhand eines voreingestellten Programms auf die Ausgabeinformation. Dabei ist es vorstellbar, dass das voreingestellte Programm veränderbar ist und insbesondere auf Erfahrungswerten basiert. Beispielsweise ist es vorstellbar, dass die Steuereinrichtung in Verbindung steht mit einem Netzwerksystem, das dazu ausgelegt ist, mittels Lernalgorithmus eine optimale Einstellung des Gasstroms in Abhängigkeit von Ausgabeinformationen vorzunehmen. Hierzu werden entsprechende Testdatensätze dem Netzwerk, insbesondere dem Lernalgorithmus, zur Verfügung gestellt. Ferner ist es vorstellbar, dass die Steuersignale in der Strömungseinrichtung stufenweise Änderungen vornehmen und/oder kontinuierliche Änderungen.

Der Patientenbereich kann wahlweise zur Untersuchung und/oder Behandlung vorgesehen sein. Dabei kann der Patient liegen, stehen und/oder sitzen. Als medizinische Vorrichtung kann vorzugsweise eine medizinische Bildgebungsvorrichtung vorgesehen sein. Zu der medizinischen Vorrichtung können auch Komponente, die zur Steuerung bzw. zum generellen Nutzen der medizinischen Vorrichtung dienen, insbesondere eine Steuereinheit und/oder Bediengeräte und/oder mechanisch bewegbare Komponenten und/oder Haltekomponenten und/oder Aufbewahrungsmittel gezählt werden. Beispielsweise kann es sich um Bildgebungsgeräte, insbesondere um ein Bildgebungsgerät für diagnostische Bildgebung und/oder für therapeutische interventionelle Bildgebung handeln. Dies kann zum Beispiel ein Magnetresonanztomograph (MRT), Röntgengerät, C-Bogen, Röntgengerät Computertomograph, PET-Gerät, Ultraschallgerät etc. sein. Das medizinische Gerät kann auch beispielsweise ein Zahnarztstuhl sein.

Die medizinische Vorrichtung ist insbesondere ein Gerät, welches nicht in Desinfektionsmittel eingelegt oder in einem Reinigungsdesinfektionsgerät, das heißt (Spülmaschine) behandelt werden kann, zum Beispiel mittels eines elektrisch betriebenen Geräts.

Vorzugsweise ist die Strömungseinrichtung Teil eines Luftkühlsystems, insbesondere einer Kühlluftumwälzung der medizinischen Vorrichtung, zum Beispiel in/um einen Tunnel und/oder in einem Bore/Röhre.

Vorzugsweise ist es hierbei vorgesehen, dass die Sensoreinrichtung dazu ausgelegt ist, eine Anzahl an Partikel im Gas zu bestimmen und/oder eine Größenverteilung der Partikel im Gas im Patientenbereich, wobei die Anzahl an Partikel oder die Größenverteilung bevorzugt die Ausgabeinformation ist. Dadurch lässt sich beispielsweise im Vergleich zu einer Normalverteilung bzw. einer Normalanzahl der Partikel im Gas feststellen, ob eine erhöhte Wahrscheinlichkeit für das Vorliegen eines Krankheitserregers in der Luft besteht. Beispielsweise wird hierzu ein optisches Messverfahren verwendet, bei dem Streulicht herangezogen wird, die Anzahl und/oder Größenverteilung der Partikel im Gas der Gasströmung festzustellen. Vorzugsweise ist die Sensoreinrichtung im Patientenbereich angeordnet und/oder in einem Luftkanal, durch den das Gas der Gasströmung durchgeführt wird. Insbesondere im Fall einer optischen Messmethode bleibt der Patient auch während einer Behandlung unbeeinträchtigt von Streulicht. Denkbar ist beispielsweise, dass das Gas unmittelbar vor Eintritt in den Patientenbereich untersucht wird und/oder nach Austritt aus dem Patientenbereich. Dabei ist es vorstellbar, dass die Sensoreinrichtung während oder nach der Anwesenheit eines Patienten im Patientenbereich ermittelt wird. Sofern eine Sensoreinrichtung eine Erhöhung der Anzahl und/oder der Abweichung der Größenverteilung während der Anwesenheit des Patienten ermittelt, kann mit einem entsprechenden Warnsignal der Nutzer und/oder der Patient darüber informiert werden, dass der Patient eventuell ein Krankheitserreger trägt. Dies kann beispielsweise als zusätzliche Diagnose herangezogen werden. Ferner ist die Sensoreinrichtung derart ausgestaltet, dass sie in Bereichen einsetzbar ist, in denen die Sensoreinrichtung hohen magnetischen Felder und/oder HF-Strahlung ausgesetzt ist.

Es ist auch vorstellbar, dass die Sensorvorrichtung dazu ausgelegt ist, eine Luftfeuchtigkeit und/oder eine Temperatur und/oder einen CO₂ -Anteil, insbesondere in einem Verhältnis zu CO, zu bestimmen. Dadurch ist es mit Vorteil möglich, weitere Parameter zu berücksichtigen, die Aufschluss geben können, ob ein Störstoff vorliegt. Außerdem lässt sich darauf rückschließen, ob ein Patient bereits im Patientenbereich ist. Hier ist es denkbar, einfach den CO₂ Gehalt zu messen, von dem man auf die Menge an verbrauchter Luft schließen kann und dadurch auch eine Filterleistung bzw. eine Luftstromleistung beeinflussen könnte.

Besonders bevorzugt ist es vorgesehen, dass die Sensoreinrichtung in einem Kanal zur Führung der Gasströmung angeordnet ist. Insbesondere ist die Sensoreinrichtung integriert in eine Luftzuführeinrichtung der medizinischen Vorrichtung. Bevorzugt umfasst die Luftzuführeinrichtung die Strömungseinrichtung und Kanäle zur Führung der Gasströmung und ist bevorzugt dazu ausgelegt, ein Gas aus dem Patientenbereich anzusaugen und vorzugsweise umzuwälzen. Dadurch kann mit Vorteil das Gas im Patientenbereich untersucht werden.

Vorzugsweise ist es vorgesehen, dass die Steuereinrichtung derart ausgelegt ist, Steuersignale in Abhängigkeit der Ausgabeinformation, insbesondere in Abhängigkeit der Anzahl an Partikel im Gas und/oder der Größenverteilung der Partikel auszugeben. Dadurch ist es vorteilhafterweise möglich, beispielsweise mit zunehmender Größe oder Anzahl an Partikeln bzw. einer zunehmenden Abweichung der Größenverteilung von einer Sollgrößenverteilung die Gasströmung entsprechend weiter zu erhöhen. Beispielsweise besteht zwischen dem Gasstromverhalten und der Abweichung von einem Sollwert und/oder einer Sollgrößenverteilung ein linearer und/oder überproportionaler Zusammenhang, sodass mit zunehmender Kontamination oder Verunreinigung des Gases die Gasströmung immer weiter erhöht werden kann, um entsprechend ausreichend und möglichst schnelle Gegenmaßnahmen auf die ermittelten Störstoffe zu veranlassen.

Vorzugsweise ist es vorgesehen, dass die die medizinische Vorrichtung eine primäre Strahlungsquelle umfasst, die für die Bildgebung, Diagnose oder die Behandlung vorgesehen ist und mit der eine variable erste Strahlungsintensität oder Energieeinstrahlung bereitstellbar ist, wobei die Steuereinrichtung derart ausgelegt ist, Steuersignale in Abhängigkeit einer aktuellen ersten Strahlungsquelle auszugeben. Die primäre Strahlungsquelle kann dabei eine Röntgenquelle, insbesondere bei einem CT-Gerät, oder ein HF-bzw. RF-Signalquelle, insbesondere bei einem MRT-Gerät, sein. Vorzugsweise ist es vorgesehen, dass dadurch in Abhängigkeit der ersten Strahlungsintensität der Gasstrom beispielsweise erhöht wird, wenn die erste Strahlungsintensität erhöht wird. Mit der erhöhten ersten Strahlungsintensität geht eine Erhöhung der Körpertemperatur des Patienten einher, die verursacht wird durch die erste Strahlungsintensität. Durch eine entsprechende Erhöhung des Gasstroms ist es dann möglich, eine Kühlwirkung durch die Gasströmung zu veranlassen, um den Komfort des Patienten weiter zu verbessern.

Weiterhin ist es bevorzugt vorgesehen, dass die medizinische Bildgebungsvorrichtung eine sekundäre Strahlungsquelle und/oder einen Filter zur Dekontamination der Gasströmung aufweist. Insbesondere handelt es sich um eine UV-Strahlungsquelle, eine DBD-Plasmavorrichtung, eine Ozonquelle oder einen HEPA-Filter, insbesondere ein wechselbarer HEPA Filter. Dadurch ist mit Vorteil eine Dekontamination möglich. Insbesondere erweist sich ein HEPA-Filter als besonders vorteilhaft für solche medizinische Bildgebungsgeräte, bei denen hohe magnetische Kräfte oder Röntgenstrahlung auftreten, da ein solcher Filter auch hier zum Einsatz kommen kann, ohne dass die Gefahr besteht, dass dieser aufgrund der magnetischen Strahlung und/oder Röntgenstrahlung beschädigt oder gar zerstört wird.

Vorzugsweise ist es auch vorstellbar, dass die medizinische Vorrichtung eine Patientenüberwachungseinrichtung aufweist. Eine Patientenüberwachungseinrichtung kann beispielsweise dazu benutzt werden, die An- oder Abwesenheit eines Patienten festzustellen. Dadurch kann beispielsweise eine Gasströmung von der Strömungseinrichtung erhöht werden, wenn kein Patient im Patientenbereich ist. Dadurch kann das Gas im Patientenbereich, das heißt die Raumluft im Patientenbereich, während der Abwesenheit eines Patienten automatisch verbessert werden. Es ist allerdings auch vorstellbar, dass mittels der Patientenüberwachungseinrichtung eine Temperatur des Patienten während der Behandlung und/oder der Untersuchung festgestellt wird und in Abhängigkeit der Körpertemperatur, die sich beispielweise aufgrund der primären Strahlungsintensität, ändert, kann dann ein Gasstrom erhöht werden, um den Komfort des Patienten zu erhöhen.

Vorzugsweise ist es vorgesehen, dass die medizinische Vorrichtung eine Warneinrichtung umfasst, die dazu ausgelegt ist, ein Warnsignal in Abhängigkeit von der Ausgabeinformation auszugeben. Beispielsweise ist es vorstellbar, dass die Sensoreinrichtung feststellt, dass die Anzahl der Partikel und/oder die Größenverteilung der Partikel einen Schwellenwert und/oder einen weiteren Schwellenwert überschreitet und damit Anzeichen dafür ist, dass ein erhöhtes Infektionsrisiko besteht. Dadurch kann nicht nur der nachfolgende Patient, sondern auch der Patient in der Untersuchungsvorrichtung über eine mögliche Krankheit oder Infektion informiert werden. Außerdem wird der behandelnde Arzt oder das behandelnde medizinische Personal geschützt, das über eine möglicherweise nicht bekannte Infektion in Kenntnis gesetzt wird. Dabei ist es vorstellbar, dass die Warneinrichtung ein Bildschirm ist oder ein Leuchtsignal ausgibt. Es ist auch vorstellbar, dass ein Duftstoff zu der Gasströmung hinzugegeben wird, um den Patienten und/oder das medizinische Personal olfaktorisch über das Überschreiten des weiteren Schwellenwertes zu informieren. Denkbar ist auch, dass zumindest ein Teil der Ausgabeinformation auf einem Bildschirm wiedergegeben wird. Dadurch kann direkt eine entsprechende Messgröße, beispielsweise der Sensorvorrichtung, abgelesen werden.

Vorzugsweise ist es vorgesehen, dass die medizinische Vorrichtung eine zusätzliche Duftstoffquelle aufweist, die dazu ausgelegt ist, dem Gasstrom ein Duftstoff oder mehrere Duftstoffe beizumischen. Dadurch ist es in vorteilhafter Weise möglich, den Komfort des Patienten im Patientenbereich zu erhöhen. Dies wirkt sich meist beruhigend auf den Patienten aus, wodurch beispielsweise auch die Aufnahmequalität verbessert werden kann, da ein flacheres Atmen und weniger Bewegung von Vorteil sind für die medizinischen Untersuchungen und insbesondere medizinische Bildgebungsverfahren.

Weiterhin ist es bevorzugt vorgesehen, dass die Steuereinrichtung ausgelegt ist, Steuersignale in Abhängigkeit einer An- oder Abwesenheit eines Patienten auszugeben. Dadurch ist es in vorteilhafter Weise möglich, die Gasströmung genau dann zu erhöhen, wenn kein Patient anwesend ist.

Ein weiterer Gegenstand der vorliegenden Erfindung ist ein Verfahren zum Betrieb einer erfindungsgemäßen medizinischen Bildgebungsvorrichtung, wobei ein Gas im Patientenbereich untersucht wird und/oder eine Gasströmung situationsabhängig mittels Steuersignale der Steuereinrichtung eingestellt wird. Alle für die medizinische Bildgebungsvorrichtung beschriebenen Vorteile und Eigenschaften lassen sich analog übertragen auf das Verfahren und andersrum.

Insbesondere ist es vorgesehen, dass die Gasströmung in Abhängigkeit von der Ausgabeinformation der Sensoreinrichtung eingestellt wird. Alternativ oder ergänzend ist es vorgesehen, dass die Gasströmung in Abhängigkeit einer ersten Strahlungsintensität der primären Strahlungsquelle eingestellt wird, wenn ein Schwellenwert überschritten wird. Dadurch kann, wie bereits beschrieben, die Kühlwirkung des Gasstroms genau dann erhöht werden, wenn ein Patient der Strahlung der primären Strahlungsquelle ausgesetzt ist. Vorzugsweise ist es vorgesehen, dass der Gasstrom in Abwesenheit eines Patienten erhöht wird. Dadurch kann beispielsweise eine Dekontamination gefördert werden in der Zeit eines Patientenwechsels.

Vorzugsweise ist es vorgesehen, dass von der Warneinrichtung ein Warnsignal ausgegeben wird, wenn die Ausgabeinformation einen weiteren Schwellenwert überschreitet. Dadurch kann das medizinische Personal und auch der Patient darüber in Kenntnis gesetzt werden, dass eventuelle eine nicht bekannte Infektion vorliegen könnte.

Der Begriff "Arbeitsplatz" bzw. Steuereinrichtung bezieht sich vorzugsweise auf einen (Personal-)Computer, eine virtuelle Maschine, die auf einer Host-Hardware läuft, einen Mikrocontroller oder einen integrierten Schaltkreis. Alternativ kann es sich bei der Steuereinrichtung um eine reale oder virtuelle Gruppe von Rechnern handeln (der Fachbegriff für eine reale Gruppe von Rechnern ist "Cluster", der Fachbegriff für eine virtuelle Gruppe von Rechnern ist "Cloud"). Vorzugsweise umfasst die Steuereinrichtung eine Recheneinheit, einen Prozessor, Schnittstellen und/oder einer Speichereinheit. Eine Recheneinheit kann Hardware- und Softwareelemente aufweisen, zum Beispiel einem Mikroprozessor oder einem Field Programmable Gate Array. Eine Speichereinheit kann als nichtpermanenter Hauptspeicher (z.B. Ran-dom Access Memory) oder als permanenter Massenspeicher (z.B. Festplatte, USB-Stick, SD-Karte, Solid State Disk) ausgeführt sein. Die Steuereinrichtung kann Teil der medizinischen Bildgebungsvorrichtung sein.

Soweit nicht bereits explizit beschrieben, können einzelne Ausführungsformen oder deren einzelnen Aspekte und Merkmale miteinander kombiniert oder ausgetauscht werden, ohne den Umfang der beschriebenen Ausführungsform einzuschränken oder zu erweitern, wenn eine solche Kombination oder ein solcher Austausch sinnvoll und im Sinne der vorliegenden Erfindung ist. Vorteile, die in Bezug auf eine Ausführungsform der vorliegenden Erfindung beschrieben sind, sind gegebenenfalls auch für andere Ausführungsformen der vorliegenden Erfindung von Vorteil, ohne den Umfang der beschriebenen Erfindung einzuschränken oder zu erweitern, wann immer eine solche Kombination oder ein solcher Austausch sinnvoll und im Sinne der vorliegenden Erfindung ist. Vorteile, die in Bezug auf eine Ausführungsform der vorliegenden Erfindung beschrieben sind, sind gegebenenfalls auch für andere Ausführungsformen der vorliegenden Erfindung von Vorteil.

Weitere Vorteile und Merkmale ergeben sich aus der nachfolgenden Beschreibung bevorzugter Ausführungsformen des erfindungsgemäßen Gegenstands mit Bezug auf die beigefügten Figuren. Einzelne Merkmale der einzelnen Ausführungsform können dabei im Rahmen der Erfindung miteinander kombiniert werden.

Es zeigt:
- **Fig. 1:**: schematisch ein medizinisches Bildgebungsgerät
- **Fig. 2:**: schematisch eine Schnittansicht durch ein medizinisches Bildgebungsgerät
- **Fig. 3:**: ein Flussdiagram zur Durchführung eines Verfahrens gemäß einer beispielhaften Ausführungsform der vorliegenden Erfindung

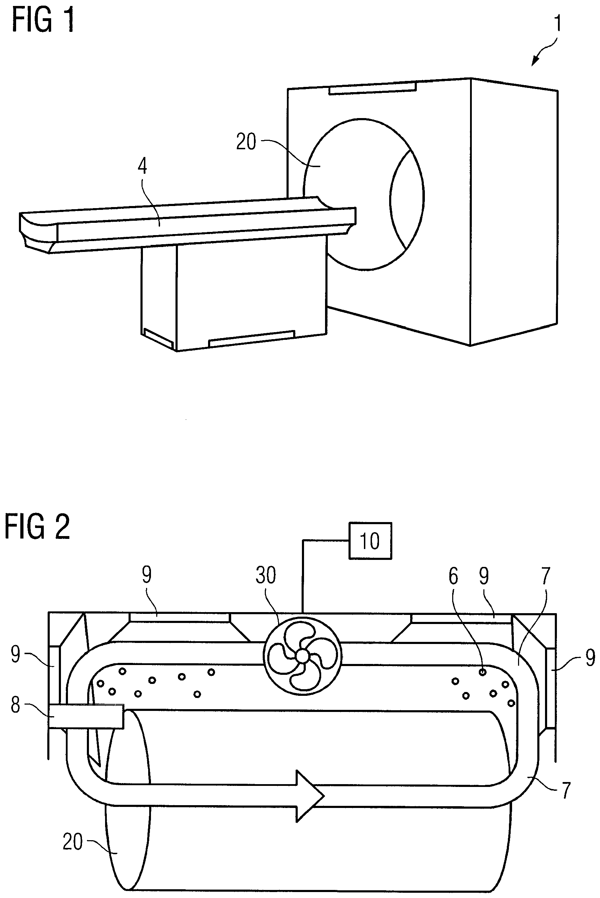

In Figur 1 ist beispielhaft eine medizinische Vorrichtung 1 dargestellt, die die vorliegende Erfindung grundsätzlich adressiert. Es handelt sich beispielsweise um ein MRT-Gerät oder CT-System, das zur medizinischen Bildgebung verwendet wird. Insbesondere handelt es sich um eine medizinische Bildgebungsvorrichtung, die dazu ausgelegt ist, ein Körperinneres eines Patienten darzustellen. Mittels einer Patientenliege bzw. Liege 4 kann ein Patient in einem Patientenbereich 20 platziert werden. In dem dargestellten Ausführungsbeispiel handelt es sich bei dem Patientenbereich 20, um den Innenbereich der rohrförmigen Ausnehmung des MRT- bzw. des CT-Systems. Die Figur 1 zeigt zwar ein MRT- bzw. ein CT-System, allerdings ist die vorliegende Erfindung nicht auf solche Bildgebungsgeräte beschränkt. Es ist auch vorstellbar, dass andere medizinische Vorrichtungen 1, die auch der Diagnose oder der Behandlung eines Patienten dienen, mit den vorteilhaften Eigenschaften der vorliegenden Erfindung ausgestattet sind.

In Figur 2 ist eine Schnittansicht durch die medizinische Vorrichtung 1 aus Figur 1 dargestellt. Insbesondere umfasst die medizinische Vorrichtung 1 eine Strömungseinrichtung 30. Die dargestellte Strömungseinrichtung 30 kann beispielsweise ein Ventilator oder ein andersartiges Luftsystem sein, das dazu ausgelegt und bestimmt ist, eine Gasströmung 7 im Patientenbereich 20 zu ändern und festzulegen. Beispielsweise lässt sich durch unterschiedliche Rotationsgeschwindigkeiten eines Ventilators eine Gasströmung 7 innerhalb des Patientenbereichs 20 beeinflussen und insbesondere einstellen. Insbesondere ist die Strömungseinrichtung 30 dazu ausgelegt, das Gas im Patientenbereich 20 umzuwälzen. Die Strömungseinrichtung 30 ist somit insbesondere dazu vorgesehen, dass eine Luft- oder Gaszirkulation in der medizinischen Vorrichtung 1 zu realisieren. Mit der Strömungseinrichtung 30 ist es vorteilhafterweise möglich, ein Volumenstrom, der durch den Patientenbereich 20 durchtritt, gezielt zu erhöhen oder zu verringern.

Besonders bevorzugt ist es vorgesehen, dass die Gasströmung 7, insbesondere die Luftströmung, erhöht wird, um einen Patienten, der im Patientenbereich 22 platziert ist, während einer Aufnahme mit dem medizinischen Bildgerät, abzukühlen. Dies erweist sich insbesondere deswegen als vorteilhaft, weil durch eine für die medizinische Bildaufnahme erforderliche Energie-Einstrahlung den Patienten erwärmt. Mittels der kühlend wirkenden Luftströmung, verursacht durch die Strömungseinrichtung 30, kann ein kühlender Effekt bewirkt werden, der den Komfort des Patienten erhöht. Besonders bevorzugt ist es vorgesehen, dass die Strömungseinrichtung 30 dazu ausgelegt ist, von einer Steuereinrichtung 10 angesteuert zu werden. Insbesondere ist die Steuereinrichtung 10 dazu ausgelegt, situationsabhängig Steuersignale auszugeben, mit denen die Strömungseinrichtung 30 ansteuerbar ist, um die Gasströmung 7 im Patientenbereich 20 situationsabhängig einzustellen. Dadurch ist es in vorteilhafter Weise möglich, eine Gasströmung 7 in Abhängigkeit beispielsweise einer Strahlungsintensität der primären Strahlungsquelle einzustellen, die die Strahlung bereitstellt, mit der die medizinische Bildaufnahme durchgeführt wird.

Beispielsweise ist es vorstellbar, dass die Gasströmung 7 erhöht wird, wenn ein Schwellenwert für die Energieeinstrahlung aus der primären Strahlungsquelle überschritten wird. Dabei ist es vorstellbar, dass die Strömungseinrichtung stufenweise oder kontinuierlich die Gasströmung 7 erhöht, insbesondere dann, wenn ein Schwellenwert der ersten Strahlungsintensität überschritten ist bzw. wird. Dadurch ist es in vorteilhafter Weise möglich, die Gasströmung 7 für solche Situationen anzupassen, in denen eine erhöhte Energieeinstrahlung, insbesondere eine HF-Leistung, dazu führt, dass der Körper eines Patienten erwärmt wird.

Es ist auch vorstellbar, dass mittels einer Temperaturmessvorrichtung eine Körpertemperatur eines Patienten beobachtet wird und anhand der gemessenen Körpertemperatur situationsabhängig die Gasströmung 7 mittels der Strömungseinrichtung erhöht wird, insbesondere, wenn die Temperatur des Patienten steigt.

Ergänzend oder alternativ ist es vorstellbar, dass die medizinische Vorrichtung 1 eine Sensoreinrichtung 25 aufweist, die dazu ausgelegt ist, das Gas der Gasströmung 7 zu untersuchen. Vorzugsweise ist die Sensoreinrichtung 25 hierzu derart in die medizinische Vorrichtung 1 eingebaut, dass die Gasströmung 7 die Sensoreinrichtung 25 passiert und die Sensoreinrichtung 25 das Gas zwecks einer Untersuchung erfassen kann. Beispielsweise ist es hierzu vorgesehen, dass zur Untersuchung und Analyse des Gases eine Anzahl an Partikel 6 und/oder eine Größenverteilung der Partikel 6 im Gas der Gasströmung 7 ermittelt und bestimmt wird. Anhand der Anzahl der Partikel 6 und oder der Größenverteilung der Partikel kann in vorteilhafter Weise darauf geschlossen werden, ob ein oder mehrere Störstoffe, wie beispielsweise Krankheitserreger, im Gas der Gasströmung 7 enthalten ist, die durch den Patientenbereich 20 geführt wird. Wird von der Sensoreinrichtung 25 festgestellt, dass die Anzahl und/oder Größenverteilung der Partikel 6 im Gas der Gasströmung 7 abweicht von einem Sollwert oder einer Sollwertverteilung, wird von der Steuereinrichtung 10 ein Steuersignal ausgegeben, mit dem die Strömungseinrichtung 10 dazu veranlasst wird, die Gasströmung zu erhöhen. Dadurch wird in vorteilhafter Weise die Gaszirkulation erhöht und die Störstoffe, insbesondere Krankheitserreger, wie COVID-19-Viren oder Grippe-Viren, aus dem Patientenbereich 20 herausbefördert.

Besonders bevorzugt ist es vorgesehen, wenn im Falle einer zirkulierenden und umgewälzten Gasströmung 7 das Gas aufgrund der erhöhten Gasströmung aus dem Patientenbereich 20 in einen Bereich geführt wird, in dem mittels geeigneter Gegenmaßnahmen einer Dekontamination des Gasstroms 7 erfolgen kann. Beispielsweise ist es jedoch hierbei vorstellbar, dass mittels eines Filters 8, beispielsweise eines HEPA -Filters, und/oder mittels sekundärer Strahlungsquellen 9, insbesondere UV-Strahlungsquellen, eine Dekontamination des Gases der Gasströmung 7 durchgeführt wird.

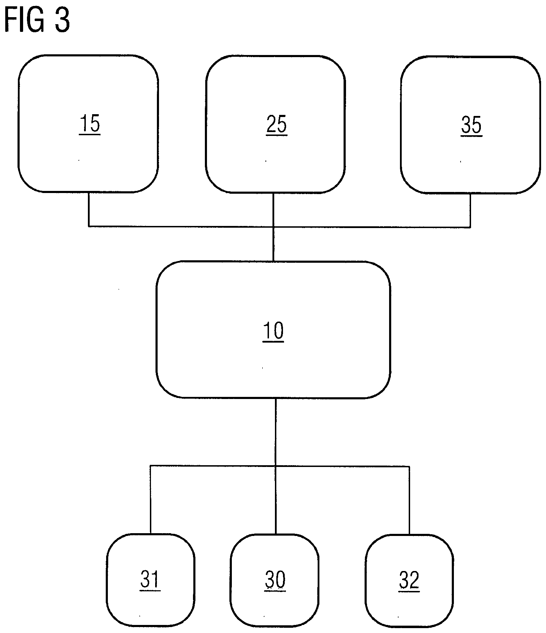

In Figur 3 ist schematisch ein Flussdiagramm zur Durchführung eines Verfahrens zum Betrieb einer medizinischen Vorrichtung 1 exemplarisch dargestellt. Insbesondere ist es hierbei vorgesehen, dass die Steuereinrichtung 10 Steuersignale ausgibt, mit denen insbesondere eine Strömungseinrichtung 10 angesteuert wird, um diese situationsabhängig in einen Zustand zu überführen, indem ein Volumenstrom des Gasströmung erhöht und/oder verringert ist. Dabei kann es sich bei der situationsabhängigen Reaktion des Steuersignals um ein Steuersignal handeln, das als Reaktion einer Ausgabeinformation einer Sensoreinrichtung 25 bereitgestellt wird. Die Sensoreinrichtung 25 gibt beispielsweise Auskunft über die Anwesenheit von Störstoffen und/oder einen Umfang an Störstoffen. Abhängig von dieser Ausgabeinformation stellt die Steuereinrichtung 10 ein Steuersignal bereit, mit dem die Strömungseinrichtung 30 eingestellt wird. Dabei ist es zum Beispiel vorstellbar, dass mit zunehmendem Umfang an Störstoffen in der Gasströmung 7 der Volumenstrom immer weiter erhöht wird, um insbesondere im Patientenbereich 20 eine Reduktion der Störstoffe zu veranlassen, und das im Patientenbereich 20 anwesende Gas auszutauschen gegen ein solches Gas, dessen Zusammensetzung weniger belastet ist durch Störstoffe.

Vorzugsweise ist hierzu vorgesehen, dass die Steuereinrichtung 10 bzw. die Sensoreinrichtung im Patientenbereich 20 angeordnet ist. Es ist allerdings auch vorstellbar, dass die Sensoreinrichtung 25 unmittelbar vor und/oder nach dem Patientenbereich, beispielsweise in einem entsprechenden Luftkanal einer Luftzirkulation, angeordnet ist.

Die Steuereinrichtung 10 kann Steuersignale bevorzugt auch in Abhängigkeit einer Lichtintensität bzw. Engergieeinstrahlung der primären Strahlungsquelle bereitstellen. Beispielsweise erhält die Steuereinrichtung hierzu Informationen über die Strahlungsintensität der primären Strahlungsquelle, die dazu herangezogen wird, eine Bildaufnahme des Patienten durchzuführen. Alternativ ist es vorstellbar, dass ein Temperaturfühler, beispielsweise eine Infrarotkamera, eine Temperatur des Patienten feststellt, anhand der ein Steuersignal festgelegt wird. Denkbar kann die Temperatur nur zu festgelegten Zeitpunkten erfolgen. Umso höher die Temperatur des Patienten im Patientenbereich 22 ist, umso höher lässt sich beispielsweise der Volumenstrom einstellen, mit dem die Gasströmung 7 durch die Patientenbereich 20 strömt. Es ist außerdem vorstellbar, dass eine Patientenüberwachungseinrichtung nicht nur eine Temperatur feststellt, sondern auch - beispielsweise mit einer Waage oder einer Kamera - feststellt, ob ein Patient im Patientenbereich anwesend oder abwesend ist. Dadurch ist es vorteilhafterweise möglich festzustellen, dass im Patientenbereich 20 zeitweise kein Patient ist. Infolgedessen lässt sich beispielsweise in Behandlungs- bzw. Bildaufnahmepausen die Gastströmung 7 mittels der Strömungseinrichtung 30 erhöhen, um dadurch wiederum einen aufbereiteten Patientenbereich 20 zur Verfügung zu stellen, der nur einen geringen Umfang an Störstoffen aufweist. Neben Krankheitserregern können Störstoffe beispielsweise auch Duftstoffe sein, die ein vorangehender Patient hinterlassen hat.

Weiterhin ist es vorstellbar, dass die Steuereinrichtung 10 mittels einer Warneinrichtung 31 ein Warnsignal ausgibt. Dies kann beispielsweise dann der Fall sein, wenn mittels der Sensoreinrichtung 25 festgestellt wird, dass die Anzahl und/oder die Größenverteilung der Partikel 6 in der Gasströmung 7 einen weiteren Schwellenwert überschritten hat. Dabei handelt sich beispielsweise bei dem weiteren Schwellenwert um einen kritischeren Schwellenwert. Dadurch wird in vorteilhafter Weise dem Nutzer und auch dem Patienten mitgeteilt, dass ein erhöhtes Infektionsrisiko besteht. In solchen Fällen kann die Strömungseinrichtung 10 den Volumenstrom erhöhen und insbesondere auch die Dauer bis zur nächsten Patientenbehandlung vergrößern.

Als Warnsignal kann beispielsweise eine akustische oder visuelle Benachrichtigung, wie beispielsweise eine Bildschirmmitteilung oder eine aufleuchtende Lampe, verwendet werden. Vorstellbar ist auch, dass die Gasströmung mittels einer Duftstoffquelle 32 ein Duftmittel oder ein Duftstoff zugeführt wird, der olfaktorisch erkennen lässt, dass ein weiterer Stellenwert überschritten wurde. Ferner ist es vorstellbar, dass die Steuereinrichtung 10 zusätzlich die Strahlung der sekundären Strahlungsquelle 9 erhöht, die dazu vorgesehen ist, die Krankheitserreger aus dem Gasstrom zu entfernen, d. h. für die Dekontamination der Gasströmung 7 zu sorgen. Denkbar ist beispielsweise, dass die Strahlungsintensität abhängig von dem Steuersignal erhöht wird und/oder eine Wellenlänge des sekundären Lichtes oder der sekundären Strahlung verschoben wird, um eine wirksamere Dekontamination zu veranlassen.

Insbesondere ist es vorgesehen, dass die Steuereinrichtung 10 dazu ausgelegt ist, einen Regelkreis zu verursachen, der abhängig von Ausgabeinformationen in der Patientenüberwachungseinrichtung 35, der primären Strahlungsquelle 15 und/oder einer Sensorreinrichtung 25 Maßnahmen ergreift, um in Echtzeit die Gasströmung 7 im Patientenbereich 20 und/oder Gegenmaßnahmen zur Aufbereitung des Gases im Patientenbereich 20 anzupassen. Zusätzlich kann der Regelkreis ergänzende Maßnahmen vorsehen, wie zum Beispiel die Ausgabe eines Warnsignals, die Zugabe von Duftstoffen und/oder die Erhöhung der sekundären Strahlungsintensität, mit der die Dekontamination des Gases in der Gasströmung 7 veranlasst wird.

## Patentansprüche

1. Medizinische Vorrichtung (1), insbesondere medizinische Bildgebungs-, Diagnose- und/oder Behandlungsvorrichtung, umfassend:
- einen Patientenbereich (20), in dem zumindest ein Teil eines Patienten platzierbar ist, und
- eine Überwachungseinrichtung, die eine Sensoreinrichtung (25) umfasst, die dazu ausgelegt ist, eine Untersuchung eines Gases im Patientenbereich (20) durchzuführen und ein Ergebnis der Untersuchung als Ausgabeinformation bereitzustellen.

2. Medizinische Vorrichtung (1) gemäß Anspruch 1, weiter umfassend
- eine Strömungseinrichtung (30), wobei die Strömungseinrichtung (30) dazu ausgelegt ist, eine Gasströmung (7) im Patientenbereich (20) zu ändern und festzulegen und
- eine Steuereinrichtung (10), die dazu ausgelegt ist, in Abhängigkeit der Ausgabeinformation der Sensoreinrichtung (25) und/oder einer weiteren Ausgabeinformation einer weiteren Überwachungseinrichtung Steuersignale auszugegeben, mit denen die Strömungseinrichtung (30) ansteuerbar ist, um die Gasströmung im Patientenbereich (20) situationsabhängig einzustellen.

3. Medizinische Vorrichtung (1) gemäß einem der vorhergehenden Ansprüche, wobei die Sensoreinrichtung (25) dazu ausgelegt ist, eine Anzahl an Partikel (6) im Gas zu bestimmen und/oder eine Größenverteilung der Partikel (6) im Gas im Patientenbereich (20) zu bestimmen, wobei die Anzahl an Partikel (6) und/oder die Größenverteilung die Ausgabeinformation ist.

4. Medizinische Vorrichtung gemäß einem der vorhergehenden Ansprüche, wobei die Sensoreinrichtung (25) dazu ausgelegt ist, eine Luftfeuchtigkeit und/oder eine Temperatur und/oder einen CO₂ -Anteil im Gas des Patientenbereich zu bestimmen.

5. Medizinische Vorrichtung gemäß einem der vorhergehenden Ansprüche, wobei die Sensoreinrichtung (25) in einem Lüftungskanal zur Führung der Gasströmung (7) außerhalb des Patientenbereichs (22) angeordnet ist.

6. Medizinische Vorrichtung (1) gemäß einem der vorhergehenden Ansprüche, wobei die medizinische Vorrichtung (1) eine primäre Strahlungsquelle umfasst, die für eine Bildgebung vorgesehen ist, und mit der eine variable erste Strahlungsintensität oder Energieeinstrahlung bereitstellbar ist, wobei die Steuereinrichtung (10) derart ausgestaltet ist, Steuersignale in Abhängigkeit der ersten Strahlungsintensität oder Energieeinstrahlung auszugeben.

7. Medizinische Vorrichtung (1) gemäß einem der vorhergehenden Ansprüche, wobei die medizinische Vorrichtung (1) zur Dekontamination der Gasströmung (7) eine sekundäre Strahlungsquelle (9) und/oder einen Filter (8) aufweist.

8. Medizinische Vorrichtung (1) gemäß einem der vorhergehenden Ansprüche, wobei die medizinische Vorrichtung (1) eine Warneinrichtung (31) umfasst, die dazu ausgelegt ist, ein Warnsignal in Abhängigkeit von der Ausgabeinformation auszugeben.

9. Medizinische Vorrichtung (1) gemäß einem der vorhergehenden Ansprüche, wobei die medizinische Vorrichtung (1) eine zusätzliche Duftstoffquelle (32) aufweist, die dazu ausgelegt ist, der Gasströmung (7) einen Duftstoff oder mehrere Duftstoffe beizumischen.

10. Medizinische Vorrichtung (1) gemäß einem der vorhergehenden Ansprüche, wobei die Überwachungseinrichtung oder eine weitere Überwachungseinrichtung eine Patientenüberwachungseinrichtung umfasst, wobei die Patientenüberwachungseinrichtung (35) als Ausgabeinformation eine Anwesenheit oder Abwesenheit des Patienten im Patientenbereich (20) bereitstellt, um an der Steuereinrichtung (10) Steuersignale in Abhängigkeit der Anwesenheit oder Abwesenheit eines Patienten auszugebenen.

11. Verfahren zum Betrieb einer medizinischen Vorrichtung (1) gemäß einem der vorhergehenden Ansprüche, wobei ein Gas im Patientenbereich untersucht wird.

12. Verfahren gemäß Anspruch 11, wobei die Gasströmung (7) situationsabhängig mittels des Steuersignals der Steuereinrichtung (10) festgelegt wird und die Gasströmung (7) in Abhängigkeit einer Ausgabeinformation einer Sensoreinrichtung (25) mittels des Steuersignals der Steuereinrichtung (10) festgelegt wird.

13. Verfahren gemäß Anspruch 12, wobei die Gasströmung (7) in Abhängigkeit einer ersten Strahlungsintensität der primären Strahlungsquelle festgelegt wird, wenn ein Schwellenwert überschritten wird.

14. Verfahren gemäß einem der Ansprüche 11 bis 13, wobei die Gasströmung (7) in Abwesenheit eines Patienten erhöht wird.

15. Verfahren gemäß einem der vorhergehenden Ansprüche, wobei ein Warnsignal ausgegeben wird, wenn die Ausgabeinformation einen weiteren Schwellwert überschreitet.
